(19)

Europäisches
Patentamt
European
Patent Office
Office européen
des brevets

(11)  **EP 4 224 143 A1**

(12)  **EUROPEAN PATENT APPLICATION**
published in accordance with Art. 153(4) EPC

(43) Date of publication:
**09.08.2023  Bulletin 2023/32**

(21) Application number: **21875540.3**

(22) Date of filing: **27.09.2021**

(51) International Patent Classification (IPC):
***G01N 21/03*** *(2006.01)*     ***G01N 21/17*** *(2006.01)*

(52) Cooperative Patent Classification (CPC):
**G01N 21/03; G01N 21/17**

(86) International application number:
**PCT/JP2021/035433**

(87) International publication number:
**WO 2022/071238 (07.04.2022 Gazette 2022/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2020  JP 2020164033**

(71) Applicant: **Sumitomo Chemical Company, Limited
Tokyo 103-6020 (JP)**

(72) Inventors:
• **OZAKI, Maya
  Niihama-shi, Ehime 792-8521 (JP)**
• **MATSUYAMA, Ryoko
  Osaka-shi, Osaka 554-8558 (JP)**

(74) Representative: **Vossius & Partner
Patentanwälte Rechtsanwälte mbB
Siebertstraße 3
81675 München (DE)**

(54)  **METHOD FOR EVALUATING EVALUATION TARGET**

(57)    A method for evaluating an evaluation target according to an embodiment includes: an irradiation step of irradiating an evaluation plate, in which an evaluation target is held in a plurality of wells provided in the evaluation plate, with illumination light from a light source; an imaging step of imaging the evaluation plate while the evaluation plate is irradiated with the illumination light in the irradiation step; and an evaluation step of evaluating the evaluation target based on an image of the evaluation plate obtained in the imaging step, wherein the evaluation target includes a tester, the plurality of wells include a test substance well for holding the evaluation target further including a test substance, in the imaging step, imaging of the whole of the evaluation plate is ended in less than 120 seconds from start of irradiation of the evaluation plate with the illumination light in the irradiation step, and irradiation with the illumination light is ended after end of imaging of the whole of the evaluation plate in the imaging step.

FIG. 4

```
        START
          |
IRRADIATE ILLUMINATION LIGHT  — S01
          |
   IMAGE EVALUATION PLATE     — S02
          |
 EVALUATE EVALUATION TARGET   — S03
          |
         END
```

**EP 4 224 143 A1**

**Description**

TECHNICAL FIELD

The present invention relates to a method for evaluating an evaluation target.

BACKGROUND ART

In biological safety evaluation, it is known to add a test substance to a tester and observe the change (for example, change in color) of the tester. For example, in the Ames test, cells which are improved not so as to synthesize amino acid by performing genetic manipulation on an amino acid synthesis gene are used as a tester. In the Ames test, a test substance is added to the cells, and then the cells are cultured under certain conditions. When mutation occurs in the amino acid synthesis gene of the cells by the test substance, the cells can synthesize amino acid again. Therefore, whether or not mutation has occurred is evaluated by confirming the presence or absence of cell proliferation. At this time, for example, when an indicator that changes in color according to the proliferation of cells is used, the proliferation of cells can be evaluated by the change in color.

In recent years, the Ames test using a plate having a plurality of wells as disclosed in, for example, Non-Patent Documents 1, 2, and 3 is known in order to reduce the amounts of tester and test substance, or the like.

PRIOR ART DOCUMENTS

NON-PATENT DOCUMENTS

Non-patent Document 1: Fluckiger-Isler S., Kamber M., "Direct comparison of the Ames microplate format(MPF) test in liquid medium with the standard Ames pre-incubation assay on agar plates by use of equivocal to weakly positive test compounds.", Mutation Research/Genetic Toxicology and Environmental Mutagenesis 747, p36-45, 2012.

Non-patent Document 2: Sui H., Kawakami K., Sakurai N., Hara T., Nohmi T., "Improvement and evaluation of high throughput fluctuation Ames test using 384-well plate with Salmonella typhimurium TA100 and TA98.", Genes and Environment 31(2), p47-55, 2009.

Non-patent Document 3: Kamber M., Fluckiger-Isler S., Engelhardt G., Jaeckh R., Zeiger E., "Comparison of the Ames II and traditional Ames test responses with respect to mutagenicity, strain specificities, need for metabolism and correlation with rodent carcinogenicity", Mutagenesis 24(4), p359-366, 2009.

SUMMARY OF THE INVENTION

PROBLEMS TO BE SOLVED BY THE INVENTION

When a plate having a plurality of wells is used, for example, the evaluation target is evaluated as follows. A plate holding an evaluation target including a tester and a test substance in each well is irradiated with an illumination light, and the plate is imaged. The color of each well is determined or the presence or absence of colonies is detected, based on the obtained image, and the evaluation target is evaluated. In this case, when the imaging condition changes during imaging of one plate, the accuracy of evaluation decreases, and thus the evaluation target cannot be appropriately evaluated.

Therefore, an object of the present invention is to provide a method for evaluating an evaluation target, the method being capable of appropriately evaluating the evaluation target.

MEANS FOR SOLVING THE PROBLEMS

A method for evaluating an evaluation target according to the present invention includes: an irradiation step of irradiating an evaluation plate, in which an evaluation target is held in a plurality of wells provided in the evaluation plate, with an illumination light from a light source; an imaging step of imaging the evaluation plate while the evaluation plate is irradiated with the illumination light in the irradiation step; and an evaluation step of evaluating the evaluation target based on an image of the evaluation plate obtained in the imaging step, wherein the evaluation target includes a tester, the plurality of wells include a test substance well for holding the evaluation target further including a test substance, in the imaging step, imaging of the whole of the evaluation plate is ended in less than 120 seconds from start of irradiation of the evaluation plate with the illumination light in the irradiation step, and irradiation with the illumination light is ended after end of imaging of the whole of the evaluation plate in the imaging step.

In the method for evaluating an evaluation target, the evaluation plate is imaged while the evaluation plate is irradiated with an illumination light. Imaging of the whole of the evaluation plate is ended in less than 120 seconds from the start of irradiation with the illumination light. Therefore, it is possible to suppress the wavelength shift (luminance change of each wavelength component) of the illumination light due to the temperature change of the light source that outputs the illumination light. As a result, the evaluation target can be accurately evaluated.

In the imaging step, imaging of the whole of the evaluation plate may be ended in 12 seconds or more and 35 seconds or less from the start of irradiation of the evaluation plate with the illumination light in the irradiation step. In this case, the wavelength shift due to the temperature change of the light source can be further reduced, so that the evaluation target can be more accurately evaluated.

The illumination light may be light having a wavelength of 520 nm or more. In this case, luminance variance for each wavelength component due to temperature change of the light source can be reduced.

In the imaging step, the evaluation plate may be imaged while the evaluation plate is conveyed.

The illumination light may be light output from an LED.

## EFFECT OF THE INVENTION

According to the present invention, it is possible to provide a method for evaluating an evaluation target, the method being capable of appropriately evaluating the evaluation target.

## BRIEF DESCRIPTION OF THE DRAWINGS

Fig. 1 is a plan view of an evaluation plate used in a method for evaluating an evaluation target according to an embodiment.
Fig. 2 is a schematic view of a cross section of a well.
Fig. 3 is a schematic view of an evaluation system used in a method for evaluating an evaluation target according to an embodiment.
Fig. 4 is a flowchart of a method for evaluating an evaluation target according to an embodiment.
Fig. 5 is a view for explaining an apparatus used in a verification experiment.
Fig. 6 shows the result of a verification experiment, and is a graph showing the relationship between the wavelength and luminance for each elapsed time from a time point at which illumination light is output.
Fig. 7 shows the result of a verification experiment, and is a graph showing the relationship between the wavelength and standard deviation for each elapsed time from a time point at which illumination light is output.
Fig. 8 shows the result of a verification experiment, and is a graph showing the temperature and the luminance difference with respect to the elapsed time from a time point at which illumination light is output.

## MODE FOR CARRYING OUT THE INVENTION

Hereinafter, embodiments of the present invention will be described with reference to the drawings. The same elements are denoted by the same reference numerals, and redundant description will be omitted. The dimensional ratios in the drawings do not necessarily coincide with those in the description.

In the present embodiment, an embodiment for evaluating safety of a test substance will be described. Specifically, the case of evaluating safety of a test substance using the Ames test will be described.

First, the outline of the Ames test used in the present embodiment will be described. In the Ames test, cells which are improved not so as to synthesize amino acid by performing genetic manipulation on an amino acid synthesis gene are used as a tester. In the Ames test, a test substance is added to the cells. Thereafter, the cells are cultured under certain conditions. When mutation occurs in the amino acid synthesis gene of the cells by the test substance, the cells can synthesize amino acid again. Therefore, whether or not mutation has occurred is evaluated by confirming the presence or absence of cell proliferation, thereby evaluating genotoxicity. Further, usually, in the Ames test, cytotoxicity evaluation is performed to confirm whether or not the test substance has toxicity to the cells.

In the present embodiment, a method for evaluating an evaluation target applicable to the Ames test will be described. In the following description of embodiments based on the Ames test, unless indicated otherwise, the tester is a cell.

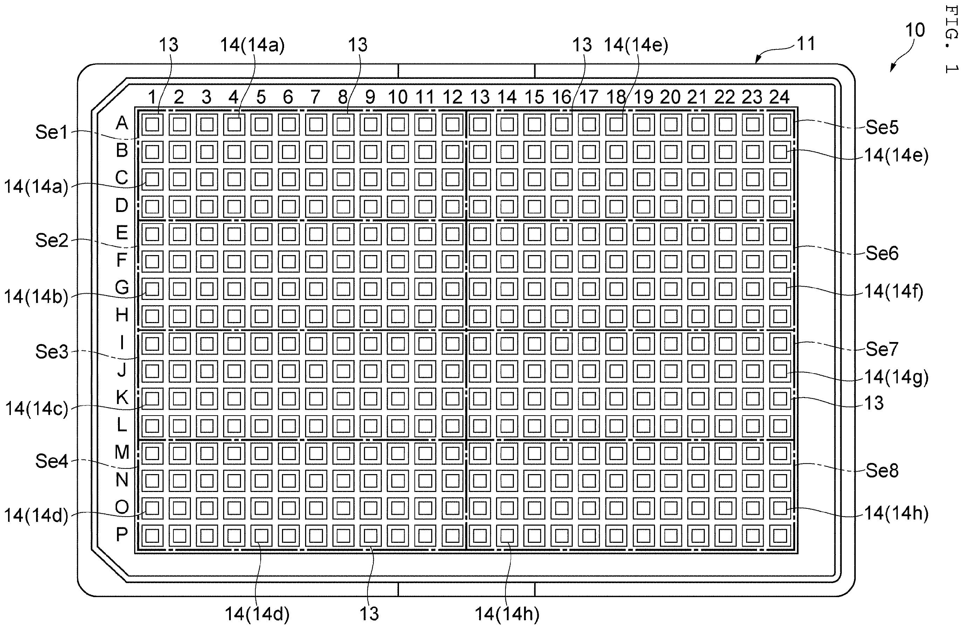

Fig. 1 is a plan view of an evaluation plate 10 used in a method for evaluating an evaluation target according to an embodiment. The evaluation plate 10 includes a plate 11. The plate 11 has a plurality of wells 13. In the present embodiment, the plate 11 has 384 wells 13 arranged two-dimensionally (16 × 24). When the plate 11 is viewed from the thickness direction, the shape of the plate 11 is a rectangular shape. In this case, an example of the length of the long side of the plate 11 is 127.0 mm to 130.0 mm, and an example of the length of the short side of the plate 11 is 85.0 mm to 87.0 mm.

In the plate 11, the first to N-th sections (N is an integer of 2 or more) are virtually set. In the present embodiment, as illustrated in Fig. 1, a case where N is 8, that is, a case where eight sections are virtually set will be described. The eight sections are hereinafter referred to as sections Se1 to Se8. The sections Se1 to Se8 are regions each including 4 × 12 (48) wells 13. The section Se1 is a solvent control section and the section Se8 is a positive control section. The solvent control section serves as a negative control section.

The well 13 is a recess (depression) formed in the plate 11. The well 13 holds an evaluation target 14. In a case where the evaluation targets 14 held in the wells 13 belonging to the respective sections Se1 to Se8 are distinguished and described, they are referred to as evaluation targets 14a to 14h.

The evaluation targets 14a to 14h each have a culture solution in which cells are dispersed (or suspended). The same amount of culture solution is held in all 384 wells 13. The amount of cells in the culture solution held in the well 13 is also the same among the wells 13. Examples of the cell include microorganisms having amino acid auxotrophic mutation and cells derived from organisms. Microorganisms having amino acid auxotrophic mutation are histidine auxotrophic *S. typhimurium* (for example, TA1535, TA1537, TA97, TA97a, TA98, TA100, TA92, TA94, TA102, TA104, TA1538, TA7001, TA7002, TA7003, TA7004, TA7005, TA7006, and various YG strains), tryptophan auxotrophic *E. coli* (for example, WP2, WP2 uvrA, WP2/pKM 101, and WP2 uvrA/pKM 101), other microorganisms, and the like. Examples of the cell derived from organisms include cultured cells derived from mammals, cells derived from other vertebrates, and cells derived from insects. The cells included in the evaluation targets 14a to 14h may be cells in which a plurality of types of cells are mixed. An example of the culture solution is a phosphate buffer containing a trace amount of amino acid.

The evaluation targets 14a to 14h further include an indicator. The amount of the indicator is the same in the evaluation targets 14a to 14h (i.e., the same in all wells 13). In the present embodiment, the indicator is a pH indicator. An example of the pH indicator is Bromocresol Purple. In the present embodiment, the color of the pH indicator is purple in the case of a negative result (in the case where no mutation has occurred in the cell), and is yellow in the case of a positive result (in the case where mutation has occurred in the cell).

The evaluation targets 14b to 14g further include a liquid (test substance solution) in which the test substance is dissolved or uniformly dispersed in a solvent, in addition to the culture solution in which the cells are dispersed and the indicator. Therefore, the wells 13 of the sections Se2 to Se7 that hold the evaluation targets 14b to 14g are wells (test substance wells) that hold the test substance. The test substance is a substance (for example, a chemical substance) whose safety (genotoxicity in the present embodiment) is to be evaluated. Examples of the solvent include water and dimethylsulfoxide (DMSO). The evaluation targets 14b to 14g are the same except that the concentration of the test substance is different. The amounts of the test substance solutions in the evaluation targets 14b of all the wells 13 belonging to the section Se2 are the same. The same applies to the sections Se3 to Se7.

The evaluation target 14a includes a solvent control solution in addition to the culture solution containing the cells and the indicator. Therefore, the wells 13 of the section Se1 holding the evaluation targets 14a are solvent control wells. The solvent control solution is a solvent used for the test substance solution. However, the solvent control solution may be any liquid that does not affect cells. Examples of the solvent control solution include water and DMSO. The amounts of the solvent control solutions in the evaluation targets 14a are the same in all the wells 13 belonging to the section Se1.

The evaluation target 14h includes a positive control solution in addition to the culture solution containing the cells and the indicator. Therefore, the wells 13 of the section Se8 holding the evaluation targets 14h are positive control wells. The positive control solution is a solution containing a positive control compound and a solvent. The positive control compound may be any compound known to be positive for cells. Examples of the positive control compound include 9AA (9-aminoacridine), 4-NQO (4-nitroquinoline-N-oxide), 2-AA (2-aminoanthracene), and 2-NF (2-nitrofluorene). The solvent need not be the same as the solvent used for the test substance solution. Examples of the solvent include water and DMSO. The amounts of the positive control solutions held in all wells 13 belonging to the section Se8 as a positive section are the same.

When the influence of the presence or absence of metabolic activation of the test substance is examined, the evaluation targets 14a to 14h further include S9 mix. S9mix is a liquid obtained by adding a coenzyme to an animal liver extract.

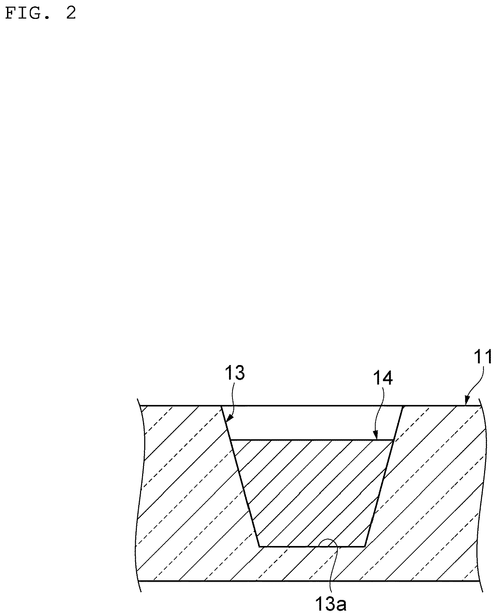

The configuration of the well 13 will be further described with reference to Figs. 1 and 2. Fig. 2 is a schematic view of a cross section of a well. Fig. 2 schematically illustrates the evaluation target 14. In the present embodiment, the plan view shape of the well 13 (the shape viewed from the depth direction of the well 13) is a square. An example of the length of one side of the opening of the well 13 is 3.0 mm to 4.0 mm. An example of the depth of the well 13 is 9.0 mm to 15.0 mm. The plate 11 having wells of such a size is known as a microplate. In an embodiment, as illustrated in Fig. 2, the well 13 is tapered toward a bottom 13a.

The evaluation plate 10 is a plate in which after the evaluation target 14 corresponding to each well 13 of the plate 11 is injected and a predetermined culture treatment is performed, the safety of the evaluation target 14 can be evaluated.

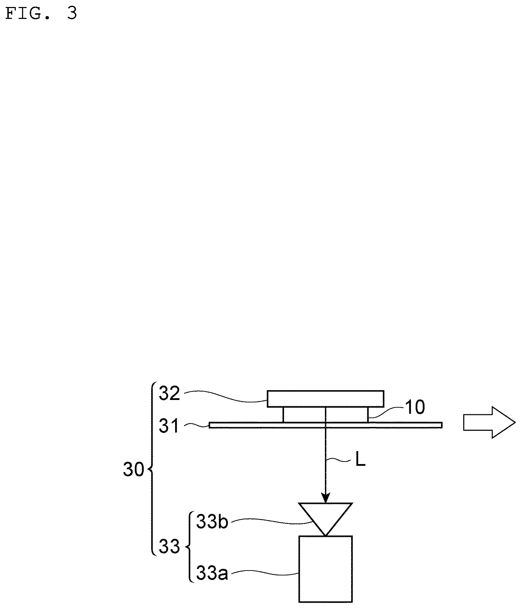

Next, an observation apparatus 30 used in the method for evaluating an evaluation target of the present embodiment will be described with reference to Fig. 3.

The observation apparatus 30 includes a stage (plate holding unit) 31, an illumination apparatus (light source) 32, and an imaging unit 33. The stage 31 supports the evaluation plate 10. The evaluation plate 10 is disposed on the stage 31 such that the bottom of the evaluation plate 10 (the bottom 13a side of the well 13) is positioned on the stage 31 side. The stage 31 is configured to transmit illumination light L output from the illumination apparatus 32 toward the imaging unit 33. For example, the material of the stage 31 may have light transmissivity, or an opening or a window portion that transmits the illumination light L may be formed in a region corresponding to a region where 384 wells 13 are formed in the evaluation plate 10. In the present embodiment, the stage 31 is configured to be conveyable in the direction of the outlined arrow in Fig. 3.

The illumination apparatus 32 is disposed on the evaluation plate 10 (the side opposite to the stage 31 as viewed from the evaluation plate 10). The illumination apparatus 32 irradiates the evaluation plate 10 with the illumination light L. An example of the illumination apparatus 32 is LED illumination, and an example of the illumination light L is light having a wavelength of 400 nm to 780 nm. In an embodiment, the illumination light L is light having a wavelength of 520 nm or more.

The imaging unit 33 receives the illumination light L transmitted through the evaluation plate 10 and images the evaluation plate 10. The imaging unit 33 includes an imaging device 33a and a light condensing unit 33b.

Examples of the imaging device 33a include two-dimensional sensors (or cameras) and line sensors. Examples of the two-dimensional sensor include CCD cameras and CMOS cameras.

The light condensing unit 33b condenses the illumination light L and causes the illumination light L to enter the imaging device 33a. The light condensing unit 33b includes at least one lens. In a case where the imaging device 33a itself has a condensing optical function, the imaging unit 33 need not include the light condensing unit 33b.

Next, an example of the method for evaluating an evaluation target according to the present embodiment will be described. Here, a case where an image of the evaluation plate 10 is acquired while the evaluation plate 10 is conveyed will be described. The conveyance speed may be determined according to, for example, a range that the frame rate and the resolution of the imaging device 33a can be actually taken, using the following equation.

$$\text{Conveyance speed [mm/s]}$$
$$= \text{length [mm] of plate 11 in conveying}$$
$$\text{direction} \div \text{imaging time [s]}$$
$$= \text{frame rate [fps] of the imaging device 33a} \times$$
$$\text{resolution [mm/pixel] of the imaging device 33a}$$

For example, in a case where the imaging time is less than 120 seconds and the resolution of the imaging device 33a is 60 um/frame or more, the evaluation plate 10 can be imaged as suitable for evaluation by the imaging device 33a having a frame rate of 19.4 fps or more when the conveyance speed is 1.17 mm/s or more. In a case where the imaging time is 12 seconds or more and less than 120 seconds and the resolution of the imaging device 33a is 8 um/frame to 60 um/frame, the evaluation plate 10 can be imaged as suitable for evaluation by the imaging device 33a having a frame rate of 19.4 fps or more when the conveyance speed is 1.17 mm/s to 11.7 mm/s. In a case where the imaging time is 12 seconds or more and 35 seconds or less and the resolution of the imaging device 33a is 15 um/frame to 25 um/frame, the evaluation plate 10 can be imaged as suitable for evaluation by the imaging device 33a having a frame rate of 160 fps or more when the conveyance speed is 4 mm/s to 11.7 mm/s. When the evaluation plate 10 is conveyed, an approach distance (for example, 30 mm) may be provided.

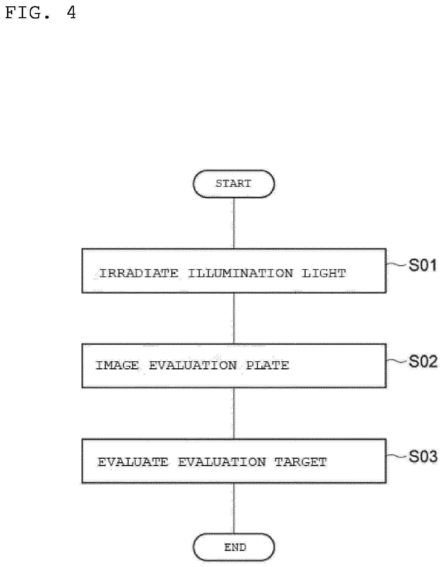

Fig. 4 is a flowchart of a method for evaluating an evaluation target according to the present embodiment. As illustrated in Fig. 4, the illumination apparatus 32 is turned on, and the evaluation plate 10 is irradiated with the illumination light L (irradiation step S01).

The evaluation plate 10 irradiated with the illumination light L is imaged by the imaging unit 33 (imaging step S02). In the imaging step S02, imaging of the whole of the evaluation plate 10 is ended in less than 120 seconds from the start of irradiation with the illumination light L in the irradiation step S01. Imaging of the whole of the evaluation plate 10 is preferably ended in 12 seconds or more and 60 seconds or less from the start of irradiation with the illumination light L, and imaging of the whole of the evaluation plate 10 is more preferably ended in 12 seconds or more and 35 seconds or less. After imaging of the whole of the evaluation plate 10 is ended, irradiation of the illumination light L is stopped. Hereinafter, the time from the start of irradiation with the illumination light L to the end of imaging of the whole of the evaluation plate 10 is also referred to as "imaging time".

In an embodiment in which the imaging area of the imaging unit 33 is smaller than the size of the evaluation plate 10, the whole of the evaluation plate 10 cannot be imaged by one imaging. In such an embodiment, the evaluation plate 10 is imaged a plurality of times so that the partial images of the evaluation plate 10 are combined to form the image of the whole of the evaluation plate 10. In this case, the imaging time is a time for completing a plurality of times of imaging of the evaluation plate 10 so that the image of the whole of the evaluation plate 10 is formed.

Next, the evaluation target 14 is evaluated based on the image captured by the imaging unit 33 (evaluation step S03). In the evaluation step S03, at least one of genotoxicity of the test substance or cytotoxicity of the test substance is evaluated. Evaluation of genotoxicity and cytotoxicity itself may be performed in accordance with the method for evaluating an evaluation target in the Ames test.

(Evaluation of genotoxicity)

In the present embodiment, when the test substance has genotoxicity to the cells included in the evaluation target 14, the color of the indicator changes from purple to yellow as a positive result. Therefore, the genotoxicity of the test substance can be evaluated by determining the color (specifically, the color of the evaluation target 14 held in each well 13) of each well 13 in the image obtained in the imaging step S02. At this time, the colors of the wells 13 in the section Se1 (solvent control section or negative control section) and the wells 13 in the section Se8 (positive control section) may be used as a reference for evaluation.

In the present embodiment, when the test substance has genotoxicity to the cells included in the evaluation target 14, there is a possibility that colonies will be generated. That is, a case where colonies are detected corresponds to a positive result. Therefore, by counting the number of wells 13 having the above colonies, the genotoxicity of the test substance can be evaluated. At this time, the states (presence or absence of colonies) of the wells 13 in the section Se1 and the wells 13 in the section Se8 may be used as a reference for evaluation.

The states (such as the concentration of the test substance) of the evaluation targets 14 held in the plurality of wells 13 in the sections Se1 to Se8 are the same. Therefore, when the evaluation target 14 is evaluated, the colors (or states) of the plurality of wells 13 included in each of the sections Se1 to Se8 may be comprehensively determined.

(Evaluation of cytotoxicity)

In the present embodiment, when the test substance has cytotoxicity to the cells included in the evaluation target 14, the transparency (specifically, the transparency of the evaluation target 14) of the well 13 changes due to the cell death or the like. Therefore, the cytotoxicity of the test substance can be evaluated based on the transparency of the well. At this time, the transparency of the wells 13 in the section Se1 (solvent control section or negative control section) may be used as a reference for evaluation. Even when the test substance is precipitated during culture, the transparency (specifically, the transparency of the evaluation target 14) of the well 13 changes. Therefore, the presence or absence of precipitation of the test substance can also be evaluated based on the transparency.

In the present embodiment, when the test substance has cytotoxicity to the cells included in the evaluation target 14, a foreign matter is generated as a result of cell death. Therefore, the cytotoxicity of the test substance can be evaluated based on the presence or absence of a foreign matter in the well. At this time, the wells 13 in the section Se1 (presence or absence of a foreign matter) may be used as a reference for evaluation.

Also in the cytotoxicity evaluation, the states (concentration of the test substance, and the like) of the evaluation targets 14 held in the plurality of wells 13 in the sections Se1 to Se8 are the same. Therefore, when the evaluation target 14 is evaluated, the transparency (or state) of the plurality of wells 13 included in each of the sections Se1 to Se8 may be comprehensively determined.

The determination of the color, the detection of colonies, the determination of the transparency, the detection of a foreign matter, and the like may be performed by the evaluator visually observing the image of the evaluation plate 10 acquired by the imaging unit 33, or may be performed by image analysis.

When the plurality of evaluation plates 10 is sequentially placed on the conveyance line and imaged, the method for evaluating an evaluation target may be applied to at least one evaluation plate 10 (for example, the second and subsequent evaluation plates 10).

In the method for evaluating an evaluation target of the present embodiment, the evaluation plate 10 is imaged while the evaluation plate 10 is irradiated with the illumination light L. At that time, the imaging of the whole of the evaluation plate 10 is ended in less than 120 seconds from the lighting start time of the illumination apparatus 32 (that is, the time point at which the irradiation with the illumination light L is started). When the imaging time is less than 120 seconds from the lighting start time of the illumination apparatus 32, it is possible to suppress the wavelength shift (luminance change of each wavelength component) of the illumination light L due to the temperature change of the illumination apparatus 32.

If the luminance of each wavelength component included in the illumination light L greatly differs between the imaging start stage and the imaging end stage of the evaluation plate 10 in association with the temperature change of the illumination apparatus 32, the color, state, and the like of the evaluation target 14 may appear to change due to the change in the imaging condition between the region imaged in the imaging start stage and the region imaged in the imaging end stage of the evaluation plate 10. As a result, the evaluation target 14 cannot be accurately evaluated.

On the other hand, when the imaging time is less than 120 seconds from the lighting start time of the illumination apparatus 32 (that is, the time point at which the irradiation with the illumination light L is started), the wavelength shift of the illumination light L due to the temperature change of the illumination apparatus 32 can be suppressed. As a result, the evaluation target 14 can be accurately evaluated. The time (imaging time) for completing the imaging of the whole of the evaluation plate 10 from the lighting start time of the illumination apparatus 32 may be 60 seconds or less, or may be 35 seconds or less. The imaging time is needs to be longer than 0 seconds, may be 5 seconds or more, or may be 12 seconds or more. In the case of 12 seconds or more, the conveyance speed can be set sufficiently slow with respect to the frame rate of the imaging device 33a. Thus, it is easy to detect a colony and a foreign matter having a certain size (for example, 60 $\mu$m) by increasing the imaging resolution. Accordingly, the imaging time may be 12 seconds or more and less than 120 seconds, 12 seconds or more and 60 seconds or less, or 12 seconds or more and 35 seconds or less.

The resolution of the imaging device 33a and the conveyance speed used in the imaging step S03 may be determined in consideration of the size of the evaluation plate 10 and the imaging time in order to appropriately detect a colony and a foreign matter having a certain size (for example, 60 $\mu$m).

A case where the size of the evaluation plate 10 is 127.7 mm $\times$ 85.6 mm, and the size of the region where the well 13 exists in the evaluation plate 10 (plate 11) is 109.5 mm $\times$ 73.5 mm will be described. In this case, examples of the resolution of the imaging device 33a and the conveyance speed according to the imaging time are as follows.

(1) A case where the imaging time is less than 120 seconds

Resolution: 60 um/frame or more
Conveyance speed: 1.17 mm/s or more
Frame rate: 19.4 fps or more

(2) A case where the imaging time is 60 seconds or less

Resolution: 60 um/frame or more
Conveyance speed: 2.33 mm/s or more
Frame rate: 38.9 fps or more

(3) A case where the imaging time is 12 seconds or more and less than 120 seconds

Resolution: 8 um/frame to 60 um/frame
Conveyance speed: 1.17 mm/s to 11.7 mm/s
Frame rate: 19.4 fps or more

(4) A case where the imaging time is 12 seconds or more and 35 seconds or less

Resolution: 15 um/frame to 25 um/frame
Conveyance speed: 4 mm/s to 11.7 mm/s
Frame rate: 160 fps or more

Under the above conditions (1) to (4), the colony or the foreign matter having a certain size, or the like can be reliably detected in the case of using an evaluation plate 10 with a size of 127.7 mm $\times$ 85.6 mm.

When the wavelength of the illumination light L is 520 nm or more, luminance variance hardly occurs. Therefore, when the illumination light L is light having a wavelength of 520 nm or more, the evaluation target 14 can be more accurately evaluated.

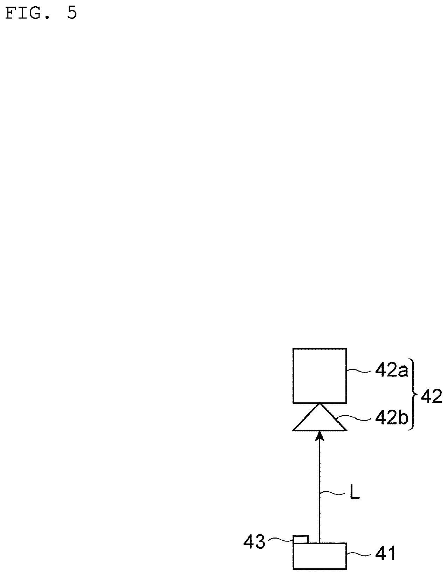

Next, a verification experiment will be described. In the verification experiment, as illustrated in Fig. 5, an imaging unit 42 was disposed facing an illumination apparatus 41. The illumination apparatus 41 was a surface light source apparatus using an LED, and was a surface light source apparatus (KDB-100, manufactured by KOS21 Company Limited) that outputs illumination light L having a wavelength of 400 to 700 nm. A temperature sensor 43 was attached to the output surface of the illumination apparatus 41. The temperature sensor 43 was AD-5625 manufactured by A&D Company, Limited. The imaging unit 42 includes an imaging device 42a and a light condensing unit 42b attached to the imaging device 42a. The imaging device 42a was a hyperspectral camera (Pika-L, manufactured by Resonon Inc.). The frame rate of the imaging device 42a was 249.2 Hz, and the exposure time was 3.93 ms. Xenoplan 1.4/23-0902 (diaphragm: F 5.6) manufactured by Jos. Schneider Optische Werke GmbH was used as the light condensing unit 42b.

In the verification experiment, data of the luminance spectrum of the illumination light L having a wavelength of 400 nm to 700 nm and the surface temperature of the illumination apparatus 41 were acquired for 30 minutes from the start of lighting of the illumination apparatus 41 (start of output of the illumination light L).

Specifically, the average value of luminance (hereinafter, referred to as "average luminance") for each wavelength at 384 locations of the surface illumination and the temperature data were acquired at intervals of 1 minute for 10 minutes from the start of lighting of the illumination apparatus 41. The average value of luminance (average luminance) for each wavelength at 384 locations of the surface illumination and the temperature data were acquired at intervals of 5 minutes after 10 minutes from the start of lighting of the illumination apparatus 41. Furthermore, the luminance at the start of lighting of the illumination apparatus 41 was set as a reference luminance. The luminance difference between the reference luminance and the average luminance, and the standard deviation σ were calculated for each wavelength. The above 384 locations were the same at the acquisition timing of each luminance data.

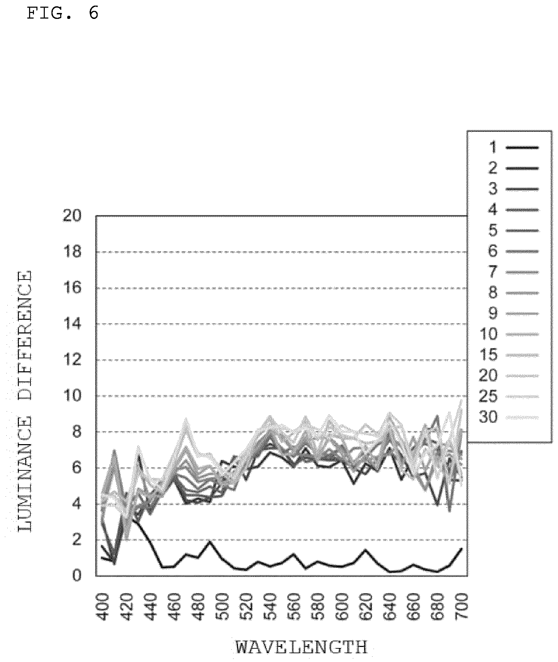

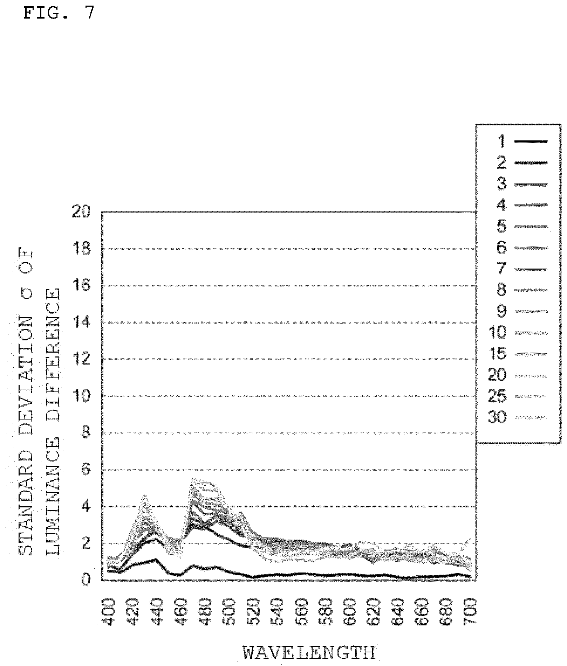

Fig. 6 is a graph showing the luminance change with respect to the wavelength, and shows data after 1 minute, 2 minutes, ..., and 30 minutes from the start of lighting by gray lines. In Fig. 6, the horizontal axis represents the wavelength (nm), and the vertical axis represents the luminance difference between the reference luminance and the average luminance. Fig. 7 is a graph showing the standard deviation with respect to the wavelength, and shows data after 1 minute, 2 minutes, ..., and 30 minutes from the start of lighting by gray lines. In Fig. 7, the horizontal axis represents the wavelength (nm), and the vertical axis represents the standard deviation of the luminance difference.

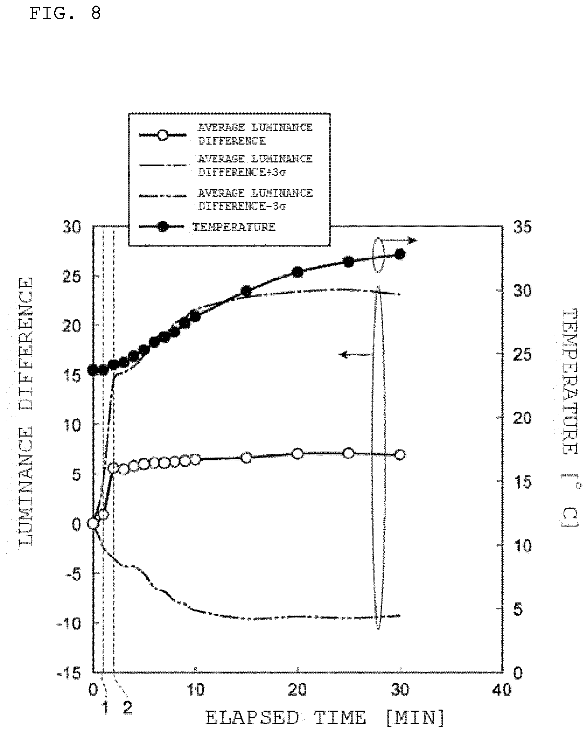

Fig. 8 is a graph showing the temperature of the illumination apparatus and the luminance change of the illumination light L with respect to the elapsed time from the start of lighting of the illumination apparatus. The horizontal axis in Fig. 8 represents the elapsed time (minutes) from the start of lighting of the illumination apparatus. The vertical axis on the right side of Fig. 8 represents the temperature, and the vertical axis on the left side represents the difference (luminance difference) between the luminance at the irradiation start time (elapsed time is 0 minute) and the average luminance of each elapsed time. The luminance difference shown in Fig. 8 is a difference between the reference luminance and the average luminance corresponding to the wavelength having the largest luminance difference among the wavelengths of 400 nm to 700 nm shown in Fig. 7 at each acquisition timing of the luminance data. The one-dot chain line in Fig. 8 represents a result obtained by adding 3σ (standard deviation) to the average luminance difference, and the two-dot chain line represents a result obtained by subtracting 3σ (standard deviation) from the average luminance difference.

As shown in Fig. 8, when the elapsed time is in less than 120 seconds (2 minutes) from the start of lighting of the illumination apparatus 41, the temperature change of the illumination apparatus 41 can be made less than 0.5°C, and the luminance change can be suppressed. As a result, it can be seen that the evaluation target 14 can be accurately evaluated when the elapsed time is less than 120 seconds. When the elapsed time is 60 seconds or less from the start of lighting of the illumination apparatus 41, the temperature change of the illumination apparatus 41 can be made less than 0.1°C. The luminance change at the time point of 60 seconds from the start of lighting of the illumination apparatus 41 is $0.93 \pm 1.10 \times 3\sigma$ (σ is standard deviation), and the luminance change can be further suppressed.

As shown in Fig. 7, when the wavelength is 520 nm or more, the luminance variance for each wavelength component in the illumination light L is small. Therefore, it is less likely to be affected by the luminance change. As a result, it can be seen that the evaluation target 14 can be accurately evaluated.

The experimental examples have been described above together with various embodiments and modifications of the present invention. However, the present invention is not limited to the various exemplified embodiments, modifications, and experimental examples, and is intended to encompass the scope indicated by the claims and to include all modifications within the meaning and scope equivalent to the claims.

The tester is not limited to cells. The test substance is not limited to a chemical substance. The tester and the test substance may be a combination in which a change (for example, change in color, change in transparency, generation of colonies, generation of a foreign matter, and the like as exemplified) serving as the determination element of safety evaluation occurs in the combination thereof. Another example of the tester is an antibody, and another example of the test substance is an antigen.

In the above embodiment, colonies were detected as a change in shape of the tester as an evaluation target, but when the shape of the evaluation target changes depending on the test result, the detection target is not limited to the colonies. The foreign matter is a substance (or a structure) different from the above-described change in shape of the tester, and for example, a substance (or a structure) that was not originally contained, and can be, for example, a substance whose boundary between the contour and the inside thereof is clear. For example, the foreign matter is a foreign matter is a substance (or a structure) separated by an interface having a refractive index different from that of the periphery of the foreign matter. Examples of the foreign matter are oil masses, bubbles, and the like.

The observation apparatus for obtaining the image of the evaluation plate may employ a reflection optical system. That is, the illumination apparatus and the imaging unit are disposed on the same side with respect to the evaluation plate, and the image of the evaluation plate may also be acquired by the imaging unit receiving the irradiation light output from the illumination apparatus and reflected by the evaluation plate.

The illumination apparatus is not limited to the LED illumination apparatus, and may be an apparatus including a halogen light source or a metal halide light source.

When the image of the evaluation plate is acquired, the evaluation plate may be imaged in a state where the evaluation plate is stationary.

Although the Ames test has been described so far, the present invention is also applicable to other biological safety evaluations involving imaging an evaluation target and evaluating safety based on the image.

The exemplified embodiments and modifications may be appropriately combined without departing from the gist of the present invention.

DESCRIPTION OF REFERENCE SIGNS

| 10 | Evaluation plate |
|----|----|
| 11 | Plate |
| 13 | Well |
| 14, 14a to 14h | Evaluation target |
| 32 | Illumination apparatus (light source) |

**Claims**

1. A method for evaluating an evaluation target, comprising:

   an irradiation step of irradiating an evaluation plate, in which an evaluation target is held in a plurality of wells provided in the evaluation plate, with an illumination light from a light source;
   an imaging step of imaging the evaluation plate while the evaluation plate is irradiated with the illumination light in the irradiation step; and
   an evaluation step of evaluating the evaluation target based on an image of the evaluation plate obtained in the imaging step,
   wherein
   the evaluation target includes a tester,
   the plurality of wells include a test substance well for holding the evaluation target further including a test substance,
   in the imaging step, imaging of the whole of the evaluation plate is ended in less than 120 seconds from start of irradiation of the evaluation plate with the illumination light in the irradiation step, and
   irradiation with the illumination light is ended after end of imaging of the whole of the evaluation plate in the imaging step.

2. The method for evaluating an evaluation target according to claim 1, wherein in the imaging step, imaging of the whole of the evaluation plate is ended in 12 seconds or more and 35 seconds or less from start of irradiation of the evaluation plate with the illumination light in the irradiation step.

3. The method for evaluating an evaluation target according to claim 1 or 2, wherein the illumination light is light having a wavelength of 520 nm or more.

4. The method for evaluating an evaluation target according to any one of claims 1 to 3, wherein in the imaging step, the evaluation plate is imaged while the evaluation plate is conveyed.

5. The method for evaluating an evaluation target according to any one of claims 1 to 4, wherein the illumination light is light output from a light-emitting diode (LED).

FIG. 1

FIG. 2

FIG. 3

FIG. 4

```
          ╭─────────╮
          │  START  │
          ╰────┬────╯
               │
   ┌───────────┴───────────────┐
   │ IRRADIATE ILLUMINATION    │──S01
   │ LIGHT                     │
   └───────────┬───────────────┘
               │
   ┌───────────┴───────────────┐
   │ IMAGE EVALUATION PLATE    │──S02
   └───────────┬───────────────┘
               │
   ┌───────────┴───────────────┐
   │ EVALUATE EVALUATION TARGET│──S03
   └───────────┬───────────────┘
               │
          ╭────┴────╮
          │   END   │
          ╰─────────╯
```

FIG. 5

FIG. 6

FIG. 7

FIG. 8

## INTERNATIONAL SEARCH REPORT

| International application No. |
|---|
| **PCT/JP2021/035433** |

**A. CLASSIFICATION OF SUBJECT MATTER**

***G01N 21/03***(2006.01)i; ***G01N 21/17***(2006.01)i
FI: G01N21/17 A; G01N21/03 Z

According to International Patent Classification (IPC) or to both national classification and IPC

**B. FIELDS SEARCHED**

Minimum documentation searched (classification system followed by classification symbols)

G01N21/03; G01N21/17

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2019-525243 A (UNIVERSITÄT DUISBURG-ESSEN) 05 September 2019 (2019-09-05) paragraphs [0010]-[0083], fig. 1-9 | 1-2, 5 |
| Y | H. SUI ET AL. IMPROVEMENT AND EVALUATION OF HIGH THROUGHPUT FLUCTUATION AMES TEST USING 384-WELL PLATE WITH SALMONELLA TYPHIMURIUM TA100 AND TA 98, GENES AND ENVIRONMENT, 2009, vol. 31, no. 2 (2009), pp. 47-55 pp. 47-51, fig. 1, 2 | 1-5 |
| Y | JP 2011-92116 A (MICROBIO KABUSHIKI KAISHA) 12 May 2011 (2011-05-12) paragraphs [0024]-[0044], fig. 1-8 | 1-5 |
| Y | WO 2018/186120 A1 (HAMAMATSU PHOTONICS KK) 11 October 2018 (2018-10-11) paragraphs [0014]-[0024], [0046], [0047], fig. 1 | 3-5 |
| Y | JP 2009-14352 A (OLYMPUS CORP.) 22 January 2009 (2009-01-22) paragraphs [0009]-[0021], [0058]-[0063], fig. 2, 21 | 4-5 |
| Y | JP 2-116735 A (SUZUKI MOTOR CORP.) 01 May 1990 (1990-05-01) pp. 3-6, fig. 1, 5 | 5 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **03 December 2021** | **14 December 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| | International application No. |
|---|---|
| | **PCT/JP2021/035433** |

**C.    DOCUMENTS CONSIDERED TO BE RELEVANT**

| Category* | Citation of document, with indication, where appropriate, of the relevant passages | Relevant to claim No. |
|---|---|---|
| A | US 2020/0131465 A1 (MOLECULAR DEVICES, LLC) 30 April 2020 (2020-04-30) | 1-5 |

**INTERNATIONAL SEARCH REPORT**
Information on patent family members

International application No.

**PCT/JP2021/035433**

| Patent document cited in search report | | | Publication date (day/month/year) | Patent family member(s) | | | Publication date (day/month/year) |
|---|---|---|---|---|---|---|---|
| JP | 2019-525243 | A | 05 September 2019 | US 2019/0162944 A1 paragraphs [0010]-[0091], fig. 1-9 WO 2018/019406 A2 DE 102016008854 A1 | | | |
| JP | 2011-92116 | A | 12 May 2011 | (Family: none) | | | |
| WO | 2018/186120 | A1 | 11 October 2018 | US 2021/0102235 A1 paragraphs [0036]-[0044], [0069], [0070], fig. 1 JP 2018-174716 A | | | |
| JP | 2009-14352 | A | 22 January 2009 | US 2010/0119132 A1 paragraphs [0066]-[0078], [0123]-[0127], fig. 2, 21 WO 2009/004984 A1 EP 2172774 A1 CN 101765773 A | | | |
| JP | 2-116735 | A | 01 May 1990 | DE 4013588 A1 columns 5-10, fig. 1, 5 | | | |
| US | 2020/0131465 | A1 | 30 April 2020 | JP 2020-522251 A WO 2018/223142 A1 | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FLUCKIGER-ISLER S. ; KAMBER M.** Direct comparison of the Ames microplate format(MPF) test in liquid medium with the standard Ames pre-incubation assay on agar plates by use of equivocal to weakly positive test compounds. *Mutation Research/Genetic Toxicology and Environmental Mutagenesis,* 2012, vol. 747, 36-45
- **SUI H. ; KAWAKAMI K. ; SAKURAI N. ; HARA T. ; NOHMI T.** Improvement and evaluation of high throughput fluctuation Ames test using 384-well plate with Salmonella typhimurium TA100 and TA98. *Genes and Environment,* 2009, vol. 31 (2), 47-55
- **KAMBER M. ; FLUCKIGER-ISLER S ; ENGELHARDT G. ; JAECKH R. ; ZEIGER E.** Comparison of the Ames II and traditional Ames test responses with respect to mutagenicity, strain specificities, need for metabolism and correlation with rodent carcinogenicity. *Mutagenesis,* 2009, vol. 24 (4), 359-366